# EUROPÄISCHE PATENTSCHRIFT

(11) **EP 2 445 473 B1**
(45) Veröffentlichungstag und Bekanntmachung des Hinweises auf die Patenterteilung: **01.02.2017**
(21) Anmeldenummer: 10725053.2
(22) Anmeldetag: 17.06.2010
(51) Int. Cl.: A61K 8/06, A61K 8/60, A61K 8/43, A61K 8/34, A61K 8/25, A61Q 5/04, A61K 8/19

(54) **HAIR RELAXER**
HAIR RELAXER
PRODUIT DÉFRISANT

(30) Priorität: 26.06.2009 DE 102009030859
(43) Veröffentlichungstag der Anmeldung: 02.05.2012
(73) Patentinhaber: Cognis IP Management GmbH, 40589 Dusseldorf (DE)
(72) Erfinder: HAAKE, Hans-Martin, 40699 Erkrath (DE); BOHLANDER, Ralf, 40699 Erkrath (DE); CECCHINI, Jessica, 40211 Düsseldorf (DE); ALTUNOK, Mehmet, 40597 Düsseldorf (DE); WEISS, Albrecht, 40764 Langenfeld (DE); BRANDS, Angela, 40667 Meerbusch (DE)
(74) Vertreter: BASF IP Association
(86) Internationale Anmeldenummer: PCT/EP2010/003650
(87) Internationale Veröffentlichungsnummer: WO 2010/149311

(56) Entgegenhaltungen:
- EP-B1- 1 242 039
- WO-A1-95/03031
- WO-A1-2009/040149
- FR-A- 1 514 179
- US-A- 3 973 574
- US-A- 5 599 531
- DATABASE WPI Week 197331 Thomson Scientific, London, GB; AN 1973-43765U XP002633673, & ZA 7 203 489 A (GILLETTE CO) 28. März 1973 (1973-03-28)

## Beschreibung

### Gebiet der Erfindung

Die Erfindung befindet sich auf dem Gebiet der chemischen Haarglättungsmittel und im besonderen auf dem Gebiet der Hydroxid-basierten Haarglättungsmittel.

### Stand der Technik

Unter Haarglättung versteht man die Entkräuselung von gelocktem Haar. Hierzu werden werden mechanische Verfahren unter Verwendung von Hitze in Form von z.B. Glätteisen in Kombination mit Haarpflegemitteln genauso verstanden wie chemische Methoden, die in die Struktur des Haares eingreifen. Bei den chemischen Haarglättungsmitteln, auch Hair Relaxer genannt, unterscheidet man zwischen Mitteln auf Basis von Thioglykolaten, also einer umgekehrten Dauerwelle, und den basischen (Lyebased) und den sogenannten nicht-basischen (No-Lye) Relaxern. Letztere verwenden Guanidiniumhydroxid und sollen weniger hautirritierend sein. Sie bestehen in der Regel aus zwei Komponenten, einer Cremebasis und einem ebenfalls basischen Aktivator, einer konzentrierten Lösung von Guanidiniumcarbonat in Wasser.

Erstere stellen Präparate auf Basis von Natrium-, Kalium- oder Lithiumhydroxiden dar. In Abhängigkeit der Haarstruktur werden unterschiedliche Hydroxidmengen in Cremes oder Gele eingearbeitet, um eine einfache Handhabung bei gleichzeitig hinreichendem Schutz der Kopfhaut zu gewährleisten.

Allen diesen chemischen Haarglättern des Standes der Technik ist jedoch gemeinsam, dass sie zum einen sehr aggressiv gegen die Kopfhaut sind und zu Irritationen führen können, zum anderen führt die Anwendung zu einer signifikanten Schädigung des Haares. Die Haare werden brüchig und spröde in einem Ausmaß, welches nicht durch anschließende Behandlung mit Konditioniermitteln ausgeglichen werden kann.

Allerdings offenbart ZA7203489 Haarglättungsmittel enthaltend 5-15 Gew. % Alkali Metal Metasilikate.

Die Aufgabe der vorliegenden Patentanmeldung bestand somit darin, chemische Haarglättungsmittel bereit zu stellen, die eine geringere Haarschädigung aufweisen als die derzeitigen Marktprodukte. Gleichzeitig sollen diese Haarglättungsmittel über eine größere Hautverträglichkeit verfügen und somit einfacher, auch durch den Endkunden, anwendbar sein.

### Beschreibung der Erfindung

Überraschenderweise wurde nun gefunden, dass Haarglättungsmittel dadurch gekennzeichnet, dass sie in einem kosmetisch akzeptablem Medium Wasserglas enthalten, wie in Anspruch 1 definiert, die oben genannten Nachteile nicht aufweisen. Bei ähnlich guter Glättungswirkung wie bei lediglich mit Alkali basisch gestellten Haarglättungsmitteln, schädigen die erfindungsgemäßen Zubereitungen die Haarstruktur deutlich weniger als diejenigen des Standes der Technik.

Wassergläser sind aus dem Schmelzfluss erstarrte, glasige, wasserlösliche Alkalisilikate (also Salze von Kieselsäuren) oder deren viskose wässerige Lösungen. Beim handelsüblichen Schmelzglas-basierten Wasserglas kommen typischerweise 1-4 Mol SiO₂ auf 1 Mol Alkalioxid (M₂O), weshalb Wasserglaslösungen üblicherweise auch durch das Gewichts- oder Molverhältnis SiO₂/Alkalioxid sowie die Dichte der wässerigen Lösung charakterisiert werden. Sie enthalten oligomere Silikatanionen mit Alkali oder quaternären Stickstoffatomen als Gegenionen (mit z.B. M = K, Na, Li, Cs, NR₄⁺). Besonders bevorzugte Wassergläser sind Natrium- oder Kaliumwassergläser. Die löslichen Silikate werden verzugsweise als wässrige Lösungen verwendet, die 15 bis 60 Gew.% Feststoffe enthalten.

Die Lösungen können sehr leicht durch weitere Basenzugaben modifiziert werden. Dabei sind für die Verwendung in kosmetischen Mitteln solche Wassergläser bevorzugt, deren molares Verhältnis von SiO₂ : M₂O (mit M=Alkali) im Bereich von 0,03 bis 2 liegt und vorzugsweise im Bereich von 0,1 bis 1 liegt. Ganz besonders bevorzugt ist der Bereich von 0,3 bis 0,8. Die löslichen Silikate gemessen als SiO₂ sind in der wässrigen Phase der erfindungsgemäßen Haarglättungsmittel in Mengen von 0,01 bis 8 Gew.%, bevorzugt 0,05 bis 5 Gew.% und besonders bevorzugt von 0,1 bis 2 Gew.% enthalten.

Herstellbar sind die Lösungen für die wässrige Phase durch Verlösen von Stückgläsern aus dem Schmelzprozeß oder durch Hydrothermale Herstellung durch Verlösung diverser denkbarer SiO₂-Quellen (Sand, Cristobalit, amorphe Kieselsäuren, Flugaschen, Kieselsolen oder Kieselgelen) in Laugen. Ebenfalls möglich ist auch ein Verlösen von hydratisiertem Alkalisilikatpulver in Wasser direkt vor Gebrauch durch den Anwender.

Die verminderte Haarschädigung stellt sich auch dann ein, wenn die erfindungsgemäßen Haarglättungsmittel neben der Wasserglas-Komponente nach separater Zugabe weitere Alkali- und/oder Erdalkalihydroxide enthalten. Somit bezieht sich ein weiterer Gegenstand der vorliegenden Erfindung auf Haarglättungsmittel, die neben Wasserglas auch Alkali- und/oder Erdalkalihydroxide, bevorzugt Natriumhydoxid, enthalten. Dabei hat sich gezeigt, dass eine Verwendung von Wasserglas : Alkali/Erdalkalihydroxid bei separater Zugabe zu den Formulierungen in einem Mengenverhältnis von 1:20 bis 5:1 besonders vorteilhaft ist.

Häufig werden die schon eingangs genannten No-Lye-Formulierungen verwendet, die Guanidincarbonate enthalten und die ein geringeres Irritations- bzw. Haarschädigungspotential aufweisen als die nur auf Alkali- oder Erdalkalimetallhydroxiden basierenden Zusammensetzungen. Der Einsatz von Wasserglas mit oder ohne weitere Zugabe von Alkali- oder Erdalkalihydroxiden führt überraschenderweise auch in den No-Lye-Formulierungen zu deutlich weniger Schädigung von Haar und Kopfhaut als in den Formulierungen ohne Wasserglas. Daher ist ein weiterer Gegenstand der vorliegenden Erfindung ein Haarglättungsmittel enthaltend Wasserglas und ggf. Alkali- und/oder Erdalkalihydroxid und Guanidincarbonat.

Die erfindungsgemäßen Haarglättungsmittel zeichnen sich weiterhin dadurch aus, dass sie als kosmetisch akzeptables Medium eine O/W-Emulsion enthalten. Diese Emulsion muss insbesondere bei alkalischen pH-Werten stabil sein, da die erfindungsgemäßen Haarglättungsmittel einen pH-Wert von vorzugsweise größer gleich 12 aufweisen. Als Emulgatoren sind daher Verbindungen vom Typ Hydroxypropylmethylcellulose, Hydroxyethylcellulose, Fettalkoholsulfate wie z.B. Natriumcetearylsulfat oder Natriumstearylsulfat oder weitere anionische Emulgatoren vom Typ Acylaminosäuren, insbesondere Natriumstearoylglutamate. Bevorzugt wird eine Emulsion auf Basis von Fettalkoholen und ethoxylierten Fettalkoholen in Kombination mit weiteren Emulgatoren.

Als Emollients können neben Fettalkoholen auch Guerbetalkohole wie z.B. Octyldodecanol oder Dialkylether wie z.B. Dicaprylylether verwendet werden.

Ein weiterer Gegenstand der vorliegenden Patentanmeldung besteht weiterhin in einem Verfahren zur Herstellung eines Haarglättungsmittels, dadurch gekennzeichnet, dass eine Lösung von Wasserglas und ggf. Alkali- und/oder Erdalkalihydroxid in das kosmetische akzeptable Medium unmittelbar vor Verwendung des Haarglättungsmittels eingerührt wird. Das kosmetisch akzeptable Medium kann dabei als O/W-Emulsion vorliegen, in die die wässrige Lösung von Wasserglas und ggf. Alkali- und oder Erdalkalihydroxid eingearbeitet wird. Die Emulsion kann jedoch auch erst durch die Einarbeitung dieser wässrigen Lösung entstehen.

Hervorragende Haarglättungsmittel lassen sich besonders einfach, somit sowohl für den Anwender als auch verfahrensökonomisch für Hersteller von Haarglättungsmitteln anwenden, wenn das Wasserglas in einem Konzentrat stabilisiert eingesetzt wird. Dieses Konzentrat muss dann nur noch während der Herstellung des Haarglättungsmittels bei der Herstellung der Emulsion zur wäßrigen Phase zugegeben werden. Somit bezieht sich diese Erfindung auch auf ein Konzentrat zur Herstellung eines Haarglättungsmittels, welches enthält:
a) 1 bis 65 Gew.%, vorzugsweise 15 bis 50 Gew.% Wasserglas,
b) 0,5 bis 80 Gew.%, vorzugsweise 5 bis 25 Gew.% Alkylpolyglykoside,
d) 0 bis 5 Gew.%, vorzugsweise 0,3 bis 0,7 Gew.% eines Komplexbildners und
e) 0 bis 10 Gew.% Natriumhydroxid.

Alkylpolyglykoside stellen bekannte nichtionische Tenside dar, die der Formel **(I)** folgen,

**R¹O-[G]ₚ** **(I)**

in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Die Alkylpolyglykoside können sich von Aldosen bzw. Ketosen mit 5 oder 6 Kohlenstoffatomen, vorzugsweise der Glucose ableiten. Die bevorzugten Alkylpolyglykoside sind somit Alkyl- und/oder Alkenyloligoglucoside. Die Indexzahl p in der allgemeinen Formel **(I)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkylpolyglykosid eine analytisch ermittelte rechnerische Größe, die meistens eine gebrochene Zahl darstellt. Vorzugsweise werden Alkylpolyglykoside mit einem mittleren Oligomerisierungsgrad p von 1,1 bis 3,0 eingesetzt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden.

Es ist jedoch auch möglich in oben genanntem Konzentrat an Stelle von Alkylpolyglykosiden andere Tenside zu verwenden. Hier seien insbesondere die folgenden mit ihren INCI-Namen und ggf. Markennamen genannt: Ethylhexylsulfat (Texapon^{®} EHS) und/oder Sodium Octyl Sulfate (Texapon^{®} 842) sowie Fettalkoholethoxylate mit Alkylkettenlängen von 6 bis 22 Kohlenstoffatomen und von 1 bis 150 Ethoxyeinheiten.

Weiterhin ist es möglich Glycerin im oben genannten Konzentrat durch Polyole zu ersetzen. Beispielhaft seien hier Zucker wie Glucose, Sorbit aber auch Naturprodukte wie Honig genannt.

Als Komplexbildner in dem oben genannten Konzentrat finden solche bevorzugt Einsatz, die bei den hohen pH-Werten der Zubereitungen stabil sind. Beispielhaft seien EDTA (Ethylendiamintetraessigsäure), NTA (Nitrilotriessigsäure), HEDP (1-Hydroxyethan-(1,1-diphosphonsäure)) und DTPA (Diethylentriaminpentaessigsäure) genannt.

### Beispiele

In der Tabelle 1 sind ein Standard Haarglättungsmittel des Standes der Technik, der lediglich Natriumhydroxid enthält, und ein erfindungsgemäßes Haarglättungsmittel enthaltend Natriumhydroxid und Wasserglas aufgeführt. Das kosmetisch akzeptable Medium ist für beide Zubereitungen identisch.

**Tabelle 1: Zusammensetzungen verschiedener Haarglättungsmittel**

| **Mengen in Gew.%** | **INCI** | **Standard Haarglättungsmittel** | **Haarglättungsmittel mit Wasserglas** |
|---|---|---|---|
| Phase A | | | |
| Emulgade 1000NI | Cetearyl Alcohol (and) Ceteareth-20 | 10 | 10 |
| Eumulgin SG | Sodium Stearoyl Glutamate | 0,2 | 0,2 |
| Lanette O | Cetearyl Alcohol | 4 | 4 |
| Paraffin | Paraffinum Liquidum | 10 | 10 |
| Vaseline | Petrolatum | 10 | 10 |

| Phase B | | | |
|---|---|---|---|
| Wasser | Aqua | Ad 100 | Ad 100 |
| Natriumhydroxid | Sodium Hydroxide (Pellets) | 2,2 | 1,5 |
| Wasserglas Kaliumsilikat | 13% SiO₂, 8% K₂O | | 7,5 |
| pH 5%ige Lösung in Wasser | | 12,4 | 12,3 |
| Erzielte Glättung (=Haarverlängerung) in % | | 18,3 | 18,8 |
| Verlust im E-Modul in % | | -57 | -28 |

Diese Formulierungen wurden auf Strähnen von gekräuseltem brasilianischem Haar aufgetragen und für 30 min so belassen. Danach wurde das Glättungsmittel ausgespült und das Haar shamponiert. Anschließend wurden die Haare getrocknet. Die erzielte Glättung wurde durch Messung der Strähnenlänge vor und nach Behandlung bestimmt. Es wurde eine Dreifachbestimmung durchgeführt. Dabei zeigte sich, dass das Wasserglas enthaltende Mittel eine leicht höhere Glättung erzielte als das andere Mittel.

Weiterhin wurde die durch die Glättungsbehandlung aufgetretene Haarschädigung über Zug/Dehnungsmessungen quantifiziert. Hierzu wurde ein vollautomatisiertes Dia-Stron MTT675 der Firma Diastron, UK verwendet. Um eine Beeinflussung der Messung durch die Luftfeuchtigkeit zu verhindern, wurden die Strähnen vor der Messung für 60 min mit entmineralisertem Wasser bedeckt und verblieben auch im Wasser während der Messung. Die Haarstärke wurde berechnet aus dem Verhältnis des Young Moduls vor und nach der Behandlung (in der Tabelle angegeben als Verlust des E-Moduls in %). Da die Messungen im Hooke'schen Bereich durchgeführt wurden, konnten die gleichen Haarfasern vor und nach der Behandlung vermessen werden.

Bei diesen Messungen zeigte sich die Überlegenheit der erfindungsgemäßen Zubereitung über die des Standes der Technik. Es tritt eine signifikant geringere Schädigung der Haare nach der Behandlung mit Wasserglas auf.

**Tabelle 2: Haarglättungsmittel hergestellt unter Verwendung eines Konzentrats:**

| **Mengen in Gew.%** | **INCI** | **Standard Haarglättungsmittel** | **Haarglättungsmittel mit Konzentrat** |
|---|---|---|---|
| Phase A | | | |
| Paraffin | Paraffinum Liquidum | 15 | 15 |
| Lanette O | Cetearyl Alcohol | 6 | 6 |
| Eumulgin B 2 | Ceteareth-20 | 2 | 2 |
| Eumulgin B 3 | Ceteareth-30 | 2 | 2 |

| Phase B | | | |
|---|---|---|---|
| Wasser | Aqua | Ad 100 | Ad 100 |
| Natriumhydroxid | Sodium Hydroxide (Pellets) | 1,8 | 1,8 |
| Konzentrat | siehe Tabelle 3 | | 5,0 |
| pH 5%ige Lösung in Wasser | | 12,2 | 12,1 |
| Erzielte Glättung (=Haarverlängerung) in % | | 100% | 100% |
| Verlust im E-Modul in % | | 75% | 53% |

Die Längenänderung wurde anders als in Tabelle 1 berechnet, da so der ursprünglichen Lockigkeit der Strähnen Rechnung getragen werden kann. Erzielte Glättung = Länge geglättete Strähne/maximale Länge der Strähne. 100 % bedeutet somit vollständige Glättung.

Das in der Tabelle 2 verwendete Konzentrat war folgendermaßen hergestellt:

**Tabelle 3: Konzentrat-Zusammensetzung**

| **Mengen in Gew.%** | **INCI** | **Compound mit Wasserglas** |
|---|---|---|
| Plantacare 810 UP | Capryl Glucoside | 11,5 |
| Perpura 60 | 29% SiO₂, 30,3% K₂O | 36,0 |
| Glycerin | Glycerin | 6,0 |
| Trilon BX | EDTA | 0,25 |
| Wasser | Aqua | Ad 100 |
| pH 5%ige Lösung in Wasser | | 12,35 |

Weiterhin wurde auch ein No-Lye-Haarglättungsmittel untersucht.

**Tabelle 4: No-Lye-Haarglättungsmittel:**

| **Mengen in Gew.%** | **Rohstoffe** | **INCI** | **Standard Haarglättungsmittel** | **Haarglättungsmittel mit Wasserglas** | **Haarglättungsmittel mit Konzentrat** |
|---|---|---|---|---|---|
| **Phase A** | | | | | |
| **1.** | Paraffinöl | Paraffinum Liquidum | 15,0 | 15,0 | 15,0 |
| | Lanette 14 | Mystristyl Alcohol | 10,0 | 10,0 | |
| | Lanette O | Cetearyl Alcohol | | | 6,0 |
| | Emulgin B2 | Ceteareth-20 | 2,5 | 2,5 | 2,0 |
| | Emulgin B3 | Ceteareth-30 | | | 2,0 |
| | | | | | |
| **2.** | Wasser | Aqua | Ad 100 | Ad 100 | Ad 100 |
| | PEG | Propylenglycol | 2,0 | 2,0 | 2,0 |
| | Lithium hydroxid | | 5,0 | 5,0 | 5,0 |
| | Wasserglas | 13% SiO₂, 8% K₂O | | 5,9 | |
| | Konzentrat | s. Tabelle 3 | | | 5,0 |
| **Phase B** | | | | | |
| | Wasser | aqua | Ad 100 | Ad 100 | Ad 100 |
| | Dekafald | DMDM Hydantoin | 0,2 | 0,2 | 0,2 |
| | (Keltrol CG-SFT) | Xantan Gum | 0,2 | 0,2 | 0,2 |
| | Guanidin Carbonate | Guanidin Carbonate | 27,0 | 27,0 | 27,0 |
| pH 5%ige Lösung der finalen Mischung in Wasser | | | 12,44 | 12,50 | 12,40 |
| Erzielte Glättung (=Haarver längerung) in % | | | 100% | 100% | 100% |
| Verlust im E-Modul in % | | | 83% | 77% | 77% |

Die Zusammensetzungen wurden derart hergestellt, dass für die Phase A die Ölkomponenten aufgeschmolzen und die Wasserkomponenten erwärmt und dann vermischt wurden. 4 Teile der Phase A wurden direkt vor der Anwendung mit 1 Teil Phase B gründlich gemischt und auf die Haare appliziert. Verlust im E-Modul wurde wie für Tabelle 1 beschrieben bestimmt, die erzielte Glättung wurde wie für Tabelle 2 beschrieben bestimmt. Es zeigt sich dabei deutlich, dass die Wasserglas enthaltenden Zubereitungen einen deutlich geringeren Verlust im E-Modul der Haare aufweisen.

## Patentansprüche

1. Haarglättungsmittel **dadurch gekennzeichnet, dass** es lösliche Alkalisilikate in einer Menge von 0,1 bis 2 Gew.-% - gemessen als SiO2 - in der wäßrigen Phase einer O/W-Emulsion enthält und einen pH-Wert größer gleich 12 aufweist.

2. Haarglättungsmittel gemäß Anspruch 1, **dadurch gekennzeichnet, dass** es neben den löslichen Silikaten zusätzlich Alkali- oder Erdalkalihydroxid enthält.

3. Haarglättungsmittel gemäß einem der Ansprüche 1 oder 2, **dadurch gekennzeichnet, dass** es Guanidincarbonat enthält.

4. Konzentrat zur Herstellung eines Haarglättungsmittels nach Anspruch 1, welches enthält:
a) 1 bis 65 Gew.% lösliche Alkalisilikate,
b) 0,5 bis 80 Gew.% Alkylpolyglykoside,
c) 0 bis 50 Gew.% Glycerin,
d) 0 bis 5 Gew.% eines Komplexbildners und
e) 0 bis 10 Gew.% Natriumhydroxid.

5. Verfahren zur Herstellung eines Haarglättungsmittels nach Anspruch 1, **dadurch gekennzeichnet, dass** eine Lösung von löslichen Alkalisilikaten und ggf. Alkali- oder Erdalkalihydroxid in eine O/W-Emulsion unmittelbar vor Verwendung eingerührt wird.

6. Verfahren zur Herstellung eines Haarglättungsmittels, **dadurch gekennzeichnet, dass** ein Konzentrat gemäß Anspruch 4 in eine Endformulierung eingerührt wird.

## Claims

1. A hair smoothing agent, **characterized in that** it comprises soluble alkali metal silicates in an amount of from 0.1 to 2% by weight - measured as SiO2 - in the aqueous phase of an O/W emulsion and has a pH greater than or equal to 12.

2. The hair smoothing agent according to claim 1, **characterized in that**, besides the soluble silicates, it additionally comprises alkali metal or alkaline earth metal hydroxide.

3. The hair smoothing agent according to one of claims 1 or 2, **characterized in that** it comprises guanidine carbonate.

4. A concentrate for the preparation of a hair smoothing agent according to claim 1, which comprises:
a) 1 to 65% by weight of soluble alkali metal silicates,
b) 0.5 to 80% by weight of alkyl polyglycosides,
c) 0 to 50% by weight of glycerol,
d) 0 to 5% by weight of a complexing agent and
e) 0 to 10% by weight of sodium hydroxide.

5. A process for the preparation of a hair smoothing agent according to claim 1, **characterized in that** a solution of soluble alkali metal silicates and optionally alkali metal or alkaline earth metal hydroxide is stirred into an O/W emulsion directly prior to use.

6. A process for the preparation of a hair smoothing agent, **characterized in that** a concentrate according to claim 4 is stirred into an end formulation.

## Revendications

1. Agent de lissage pour cheveux, **caractérisé en ce qu'**il contient des silicates de métal alcalin solubles en une quantité de 0,1 à 2% en poids - mesurés sous forme de SiO₂ - dans la phase aqueuse d'une émulsion H/E et présente un pH supérieur à 12.

2. Agent de lissage pour cheveux selon la revendication 1, **caractérisé en ce qu'**il contient en plus, outre les silicates solubles, un hydroxyde de métal alcalin ou alcalino-terreux.

3. Agent de lissage pour cheveux selon l'une quelconque des revendications 1 ou 2, **caractérisé en ce qu'**il contient du carbonate de guanidine.

4. Concentrat pour la préparation d'un agent de lissage selon la revendication 1 qui contient
a) 1 à 65% en poids de silicates de métal alcalin solubles,
b) 0,5 à 80% en poids de polyglucosides d'alkyle,
c) 0 à 50% en poids de glycérol,
d) 0 à 5% en poids d'un complexant et
e) 0 à 10% en poids d'hydroxyde de sodium.

5. Procédé pour la préparation d'un agent de lissage pour cheveux selon la revendication 1, **caractérisé en ce qu'**une solution de silicates de métal alcalin solubles et le cas échéant d'hydroxyde de métal alcalin ou alcalino-terreux est délayée dans une émulsion H/E juste avant l'utilisation.

6. Procédé pour la préparation d'un agent de lissage pour cheveux, **caractérisé en ce qu'**un concentrat selon la revendication 4 est délayé dans une formulation finale.
